# EUROPEAN PATENT APPLICATION

(11) **EP 4 696 236 A1**
(43) Date of publication of application: **18.02.2026**
(21) Application number: 24788528.8
(22) Date of filing: 18.03.2024
(51) Int. Cl.: A61B 5/16

(54) **INFORMATION PROCESSING SYSTEM, INFORMATION PROCESSING DEVICE, AND INFORMATION PROCESSING METHOD**

(30) Priority: 12.04.2023 JP 2023065126
(71) Applicant: Stolia Co., Ltd., Tokyo 104-0061 (JP); MITSUBISHI PENCIL COMPANY, LIMITED, Tokyo 140-8537 (JP); The University Of Tokyo, Tokyo 113-8654 (JP)
(72) Inventor: TATANI Keiji, Tokyo 104-0061 (JP); FUKUDA Takamasa, Tokyo 140-8537 (JP); IKEGAYA Yuji, Tokyo 113-8654 (JP)
(74) Representative: Guardian IP Consulting I/S
(86) International application number: PCT/JP2024/010562
(87) International publication number: WO 2024/214498

(57) **Abstract**

Provided are an information processing system, an information processing apparatus, and an information processing method for predicting biological indicators, such as brain waves and heart rate, and biological behaviors, such as eye movements and blinking, from a writing motion. One aspect of the present disclosure provides an information processing system including an electronic device and an information processing device. The electronic device includes an acceleration detection unit configured to detect information regarding acceleration during the writing motion and a transmission unit configured to transmit the information regarding acceleration detected by the acceleration detection unit to the information processing device. The information processing device includes a reception unit configured to receive the information regarding acceleration transmitted from the transmission unit and a prediction processing unit configured to output information regarding a prediction for a biological indicator or a biological behavior of a user based on a pre-established artificial intelligence learning model, using the information regarding acceleration received by the reception unit as input.

## Description

### TECHNICAL FIELD

The present disclosure relates to an information processing apparatus, an information processing apparatus, and an information processing method for predicting brain waves based on body movements.

### BACKGROUND

Brain waves are electrical signals representing electrical activities generated by a brain and are classified into several types according to their frequencies. Various brain activities and physical conditions can be read from the brain waves, but there are still many unknown aspects.

Patent document 1 discloses an electroencephalograph that allows an electrode to contact a predetermined position on the head for accurately measuring brain waves. The electroencephalograph is a wearable electroencephalograph for a head, including: an arch-shaped main frame extending along the head toward left and right auricles when the electroencephalograph is worn on the head; an electrode unit being provided on the main frame and having a pair of electrodes for detecting brain waves; and wearable units, provided at respective ends of the main frame along its extensional direction, worn on the left and right auricles, and having protrusions inserted into left and right ear canals.

Patent document 2 discloses a brain wave measuring device capable of performing stable measurement of brain waves in daily life without causing discomfort to surrounding people. The brain wave measuring device includes: a measuring device main body that can be attached to the head of a user; a brain wave electrode, connected to the measuring device main body, disposed at a brain wave detecting position of the user, and configured to detect brain wave signals of the user; and an electrode position adjusting mechanism portion, configured to adjust a relative position of the brain wave electrode with respect to the measuring device main body.

Patent document 3 discloses a method for accurately measuring degree of empathy between test subjects based on their brain waves. In this method for measuring degree of empathy, a computer acquires first brain wave data from a first subject and second brain wave data from a second subject, where the first brain wave data and the second brain wave data are synchronized with an accuracy of less than 1000 microseconds. A correlation between the first brain wave data and the second brain wave data are calculated. The degree of empathy regarding content between the first and second subjects is estimated based on the calculated correlation.

Patent document 4 discloses an estimating device configured to estimate degree of attraction instinctively or intuitively generated when facing a product. The estimating device includes: a brain wave data acquiring unit, configured to acquire brain wave data from a subject within 1000 milliseconds after at least one of the subject's five senses is stimulated by an object; an event-related potential acquiring unit, configured to acquire an event-related potential from the brain wave data; and an estimating unit, configured to estimate the degree of attraction based on the event-related potential.

### Prior Art Reference

### Patent Documents

Patent Document 1: Japanese Patent Application Publication No. 2019-000405
Patent Document 2: Japanese Patent Application Publication No. 2022-077070
Patent Document 3: Japanese Patent Application Publication No. 2022-155614
Patent Document 4: Japanese Patent Application Publication No. 2022-160124

### SUMMARY

### Problems to be solved

For example, when studying by performing a writing process with a writing tool, a person performing a writing motion is not always concentrating. The writing motion may stagnate, concentration may be dispersed, or the person may become idle, thereby failing to perform the intended writing or studying motion. Movements during states of concentration and those during non-concentrating states differ among individuals, making objective measurement difficult. Although it is conceivable to obtain biological indicators such as brain waves and heart rate reflecting concentration, acquiring such indicators requires specialized equipment. In addition, biological behaviors such as reduced eye movement and decreased blink frequency during states of concentration can only be measured by using cameras or eyeglass-type devices. Accordingly, it is difficult to measure concentration objectively, and there is a need for a device capable of easily and objectively measuring concentration, notifying the user when concentration lapses, and prompting the user to take actions for enhancing concentration.

The present disclosure aims to provide an information processing system, an information processing apparatus, and an information processing method for predicting biological indicators, such as brain waves and heart rate, and biological behaviors, such as eye movements and blinking, from a writing motion.

### Means for solving the Problems

One aspect of the present disclosure provides an information processing system including an electronic device and an information processing device. The electronic device includes an acceleration detection unit configured to detect information regarding acceleration during the writing motion and a transmission unit configured to transmit the information regarding acceleration detected by the acceleration detection unit to an information processing device. The information processing device includes a reception unit configured to receive the information regarding acceleration transmitted from the transmission unit and a prediction processing unit configured to output information regarding a prediction for a biological indicator or a biological behavior of a user based on a pre-established artificial intelligence learning model, using the information regarding acceleration received by the reception unit as input.

Another aspect of the present disclosure provides an information processing apparatus including an acceleration detection unit and a prediction processing unit. The acceleration detection unit is configured to detect information regarding acceleration during a writing process. The prediction processing unit is configured to use the information regarding the acceleration detected by the acceleration detection unit as input, and to output information regarding a prediction for a biological indicator or a biological behavior of a user based on a pre-established learning model of artificial intelligence.

Another aspect of the present disclosure provides an information processing method including an acceleration acquisition step and a prediction processing step. In the acceleration acquisition step, information regarding acceleration during a writing motion is acquired. In the prediction processing step, the information regarding the acceleration acquired in the acceleration acquisition step is used as input, and information regarding a prediction for a biological indicator or a biological behavior of a user is output based on a pre-established artificial intelligence learning model.

According to the configuration of the information processing system, the information processing apparatus, and the information processing method, information regarding acceleration during a writing motion is used as input, and information regarding a prediction for a biological indicator or a biological behavior of a user is output based on a pre-established artificial intelligence learning model. In other words, the biological indicator or the biological behavior of the user is predicted from the information regarding acceleration during the writing motion by an artificial intelligence that uses the correlation between the information regarding acceleration during the writing motion and a biological indicator or a biological behavior of the user as a learning model,.

### Effects

According to the present disclosure, it is possible to provide an information processing system and an information processing method that predict biological indicators such as brain waves and heart rate, and biological behaviors such as eye movements and blinking, from a writing motion.

### BRIEF DESCRIPTION OF DRAWINGS

Fig. 1 is a block diagram illustrating the configuration of an information processing system in accordance with the present disclosure.
Fig. 2 is a schematic diagram illustrating a specific example of the information processing system in accordance with the present disclosure.
Fig. 3 is a block diagram illustrating the configuration of an information processing apparatus in accordance with the present disclosure.
Fig. 4 is a flowchart illustrating a specific example of an information processing method in accordance with the present disclosure.
Fig. 5 is a diagram illustrating Arabic characters used in an experiment.
Fig. 6 is a diagram illustrating a relationship between brain wave data predicted from acceleration and actually measured brain wave data.
Fig. 7 is a diagram illustrating a relationship between brain wave data predicted from acceleration for multiple subjects and actually measured brain wave data.
Fig. 8 is a diagram illustrating a relationship between brain wave data predicted by a learning model at one-minute intervals and actually measured brain wave data.
Figs. 9A and 9B are diagrams illustrating a comparison between brain waves predicted from a writing motion and actual brain waves.
Figs. 10 A and 9B are diagrams illustrating a comparison between brain waves predicted from a writing motion and actual brain waves.

### DETAILED DESCRIPTION

Hereinafter, embodiments of the present disclosure will be described with reference to the drawings. In the following description, same reference numerals are assigned to the same components, and a description of a previously explained component may be omitted as appropriate.

### (Configuration of the Information Processing System)

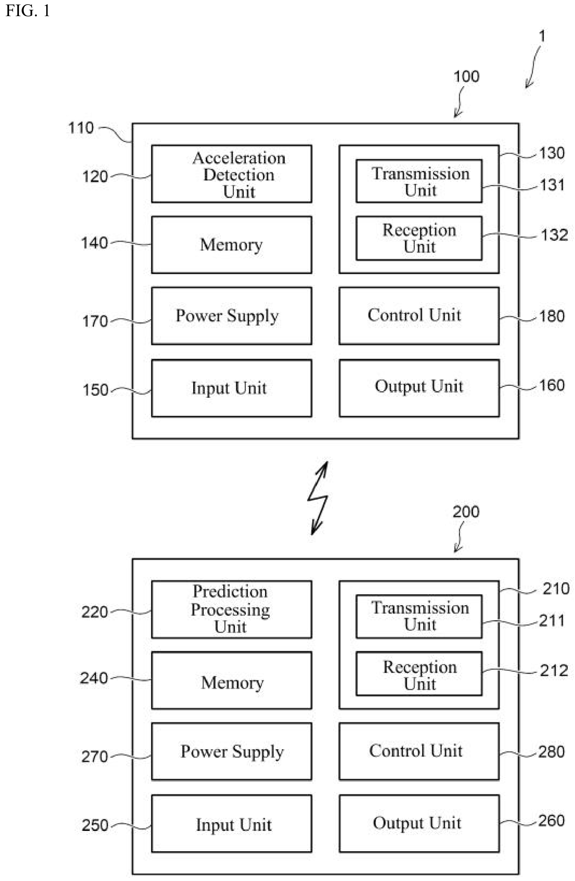

FIG. 1 is a block diagram illustrating the configuration of the information processing system in accordance with the present disclosure.

The information processing system 1 according to the present disclosure is, for example, a system that predicts biological indicators reflecting concentration, such as brain waves and heart rate, from a writing motion performed when using a writing instrument.

As shown in FIG. 1, the information processing system 1 includes an electronic device 100 and an information processing device 200. The electronic device 100 includes a main body 110, an acceleration detection unit 120, and a transmission unit 131. The main body 110 may be implemented as, for example, a pen-like shape having a writing function, or may be configured to be attachable to a writing instrument.

The acceleration detection unit 120 is a part configured to detect information regarding acceleration during a writing motion (hereinafter also referred to as "acceleration information"). The acceleration detection unit 120 includes, for example, an acceleration sensor. In addition to the acceleration sensor, the acceleration detection unit 120 may further include a tilt sensor, a rotational trajectory sensor, a gravitational acceleration sensor, a gyroscope sensor, an inertial sensor, or a pressure sensor. The acceleration detection unit 120 may also detect acceleration information from a writing motion captured by a camera. The acceleration information may include, in addition to a value of the acceleration itself, information derived from the acceleration (such as velocity, angular change, angular velocity, and rotational angle change). Furthermore, the acceleration detection unit 120 may be provided either inside or outside the main body 110.

The transmission unit 131 is a part configured to transmit the acceleration information detected by the acceleration detection unit 120 to the information processing device 200. The transmission unit 131 may be configured to be included in a communication unit 130 together with a reception unit 132. Information transmission and reception by the communication unit 130 may be wireless communication or wired communication.

In addition to the above configuration, the electronic device 100 may further include a memory 140, an input unit 150, an output unit 160, a power supply 170, and a control unit 180. The memory 140 is, for example, a non-volatile storage device. The input unit 150 is, for example, a switch, a button, a touch panel, a camera, a microphone, or the like. The output unit 160 is, for example, a speaker, a lamp, a vibration device, or the like. The power supply 170 is, for example, a storage battery. The control unit 180 is, for example, a CPU that controls the respective units.

The information processing device 200 includes a receiving unit 212 and a prediction processing unit 220. The receiving unit 212 is a part configured to receive the acceleration information transmitted from the transmission unit 131 of the electronic device 100. The receiving unit 212 may be configured to be included in a communication unit 210 together with a transmission unit 211. Information transmission and reception by the communication unit 210 may be wireless communication or wired communication.

The prediction processing unit 220 is configured to use the acceleration information received by the receiving unit 212 as input and to output information regarding a prediction for a biological indicator of a user based on a pre-established learning model of artificial intelligence. For example, the prediction processing unit 220 uses the learning model based on a correlation between the acceleration information and brain waves during the writing motion. In addition, the prediction processing unit 220 may use the learning model based on a correlation between a temporal variation pattern of the acceleration during the writing motion and the biological indicator representing any one of a degree of concentration, a degree of relaxation, or a degree of comfort of the user performing the writing motion.

The information processing device 200 may further include, in addition to the above configuration, a memory 240, an input unit 250, an output unit 260, a power supply 270, and a control unit 280. The memory 240 is, for example, a non-volatile storage device. The input unit 250 is, for example, a switch, a button, a touch panel, a camera, a microphone, or the like. The output unit 260 is, for example, a speaker, a lamp, a vibration device, or the like. The power supply 270 is, for example, a storage battery. The control unit 280 is, for example, a CPU that controls the respective units.

For example, a mobile terminal (a smartphone, a tablet PC (Personal computer)) or a computer is used for the information processing device 200.

In the information processing system 1 according to the present disclosure, the electronic device 100 acquires acceleration information during the writing motion and transmits the information to the information processing device 200. The information processing device 200 receives the acceleration information transmitted from the electronic device 100 and inputs it to the prediction processing unit 220. The prediction processing unit 220 takes the acceleration information during the writing motion as input and outputs information regarding a prediction for the biological indicator of the user based on a pre-established artificial intelligence learning model. That is, in the information processing system 1, the biological indicator of the user can be predicted from the acceleration information during the writing motion by the artificial intelligence that uses the correlation between the acceleration information during the writing motion and the biological indicator of the user as a learning model.

### (A Specific Embodiment of the Information Processing System)

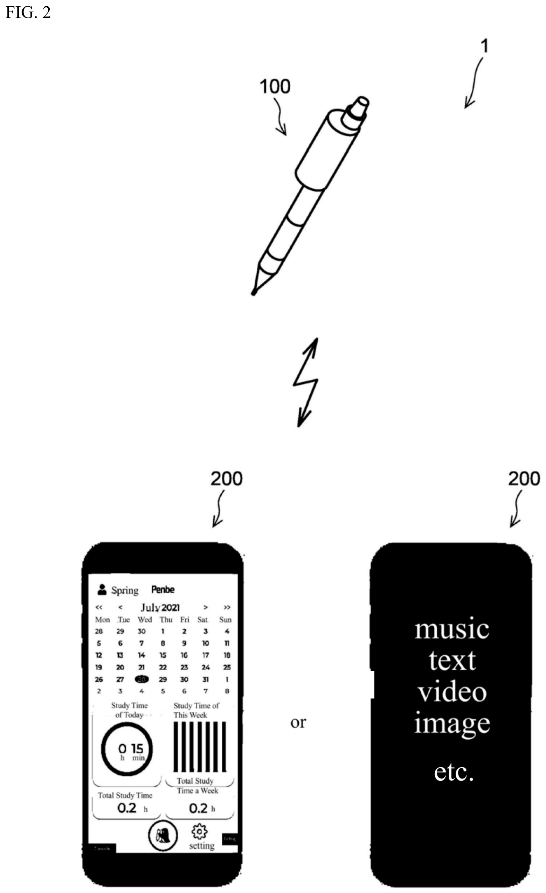

FIG. 2 is a schematic diagram illustrating a specific example of the information processing system according to the present disclosure.

As a specific example of the electronic device 100 of the information processing system 1, a writing instrument or an attachment type as following is applied. The writing instrument is equipped with an acceleration sensor, a pressure sensor for detecting pen pressure, and communication functions such as Bluetooth^{®} or Wi-Fi^{®}. The attachment type is an attachment attached to a writing instrument and has an acceleration sensor, a pressure sensor, and communication functions such as Bluetooth^{®} or Wi-Fi^{®}.

As a specific example of the information processing device 200 of the information processing system 1, a smartphone or a tablet PC having communication functions such as Bluetooth^{®} or Wi-Fi^{®} is applied. The prediction processing unit 220 of the information processing device 200 may be implemented as application software executed on a smartphone or a tablet PC, or as software executed on an external server connected to a network.

In the electronic device 100, by using AI (artificial intelligence) or machine learning, the acquired acceleration data and pressure data are used to analyze the user's concentration state, and to predict the user's concentration state, concentration time, and study time. Based on the predicted concentration state, concentration time, and study time, the information processing device 200, such as the smartphone or tablet PC, outputs at least one of sound, image, video, color, or text so as to create (or promote) a state in which the user can maintain sustained concentration.

### (Configuration of the Information Processing Apparatus)

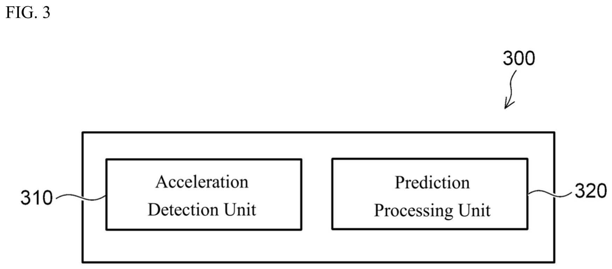

FIG. 3 is a block diagram illustrating the configuration of the information processing apparatus in accordance with the present disclosure.

The information processing apparatus 300 according to the present disclosure is an apparatus configured to predict, for example, the biological indicator representing concentration, such as brain waves or heart rate, based on the writing motion performed using a writing instrument. For example, a mobile terminal (a smartphone, a tablet PC) or a computer is used for the information processing apparatus 300.

The information processing apparatus 300 includes an acceleration detection unit 310 and a prediction processing unit 320. The acceleration detection unit 310 is configured to detect acceleration information in the writing motion. The prediction processing unit 320 is configured to output information regarding a prediction for a biological indicator of a user based on the acceleration information detected by the acceleration detection unit 310. Here, the acceleration detection unit 310 and the prediction processing unit 320 constituting the information processing apparatus 300 may be configured to be provided within a physically device, or may be configured to be provided physically separate and connected via a network.

The acceleration detection unit 310 is a part configured to detect the acceleration information in the writing motion. The acceleration detection unit 310 detects the acceleration information based on an amount of change in velocity per unit time during the writing motion. A means for reading the writing motion (reading means) may be provided inside the information processing apparatus 300 or may be provided externally. For example, the acceleration information is detected from a writing motion along handwriting or a trajectory on a touch panel (an example in which the reading means is provided inside), or a writing motion performed within a predetermined space is read by a camera or a motion sensor attached to the hand (an example in which the reading means is provided externally), and the acceleration information is detected from the read information.

The prediction processing unit 320 uses the acceleration information detected by the acceleration detection unit 310 as input, and outputs information regarding a prediction for a biological indicator of the user based on a pre-established artificial intelligence learning model. For example, the prediction processing unit 320 uses the learning model based on a correlation between the acceleration information and brain waves during the writing motion. In addition, the prediction processing unit 320 may use the learning model based on a correlation between a temporal variation pattern of the acceleration during the writing motion and the biological indicator representing any one of a degree of concentration, a degree of relaxation, or a degree of comfort of the user performing the writing motion. The prediction processing unit 320 may be implemented as application software executed on a smartphone or tablet PC, or as software executed on an external server connected to a network.

In such information processing apparatus 300, the acceleration information during the writing motion detected by the acceleration detection unit 310 is used as input to the prediction processing unit 320, and the information regarding a prediction for a biological indicator of the user is output based on the pre-established artificial learning model of intelligence. That is, in the information processing apparatus 300, the biological indicator representing concentration, such as the brain wave or the heart rate of the user, can be predicted from the acceleration information during the writing motion by using the artificial intelligence that uses the correlation between the acceleration information during the writing motion and the biological indicator of the user as a learning model.

### (Information processing method)

Subsequently, the information processing method according to the present disclosure will be described.

The information processing method according to the present disclosure includes an acceleration acquisition step and a prediction processing step. In the acceleration acquisition step, information regarding acceleration during a writing motion is acquired. In the prediction processing step, the information regarding the acceleration acquired in the acceleration acquisition step is used, and information regarding a prediction for a biological indicator or a biological behavior of a user is output based on a pre-established artificial intelligence learning model.

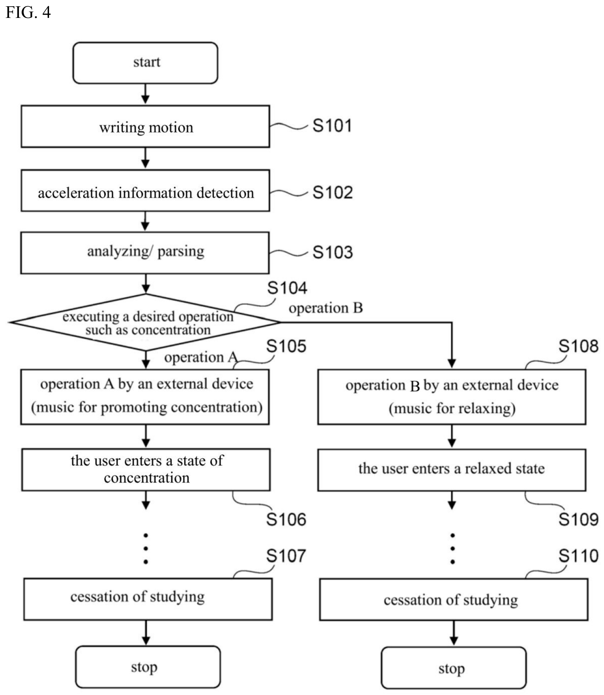

FIG. 4 is a flowchart illustrating a specific example of an information processing method in accordance with the present disclosure.

First, a user writes characters, numbers, illustrations, pictures, or the like on a paper teaching material or tablet, or the like (step S101). At that time,, information such as acceleration information, pen pressure, pen strokes, and the like is acquired from a writing instrument itself, an attachment mounted on the writing instrument, a stylus pen, or a touch screen itself (step S102: acceleration acquisition step).

Next, based on the acquired information such as acceleration information, pen pressure, pen strokes, and the like, it is analyzed and determined whether the user is concentrating, that is, whether the brain generates brain waves representing concentration (step S103: prediction processing step). Artificial intelligence or machine learning techniques are adopted for the analysis and determination. During the determination, the brain waves are associated with the acceleration information during the writing motion, and a prediction model/a discrimination model is prepared using the artificial intelligence or machine learning. Additionally, it is also possible to use a learning model based on a correlation between a temporal variation pattern of the acceleration during the writing motion and the biological indicator representing any one of a degree of concentration, a degree of relaxation, or a degree of comfort of the user performing the writing motion.

In this way, simply holding the writing instrument or performing the writing motion enables the user's brain wave state or concentration state to be determined or predicted without using any special measuring instruments such as an electroencephalograph.

Subsequently, based on information such as the concentration state determined or predicted in step S103, it is determined whether a desired operation is executed (step S104). In the present embodiment, as an example of the operation to be executed, one operation is selected from operation A and operation B. In addition, it is noted that any one of three or more operations may also be selected.

For example, if it is determined, based on information such as the concentration state determined/predicted in step S103, that the user is not concentrating well, operation A is executed. In the operation A, first, an external device (the information processing device 200 or another device) is made to execute an operation for prompting concentration (step S105). For example, music or the like that can promote concentration is played by the external device. If the user thereby enters a state of concentration (step S106), an operation for prompting cessation of studying is executed after a predetermined time period (step S107). In order to prompt the cessation of studying, the external device may play an alarm sound or display, on a screen, a notification for stopping studying.

On the other hand, if it is determined, based on information such as the concentration state determined/predicted in step S103, that the user has maintained a state of concentration for a certain period, the operation B is executed. In the operation B, first, an external device is made to execute an operation for prompting relaxation (step S108). For example, music that can promote relaxation is played by the external device. If the user thereby enters a relaxed state (step S109), an operation for prompting cessation of studying is executed after a predetermined time period (step S110). In order to prompt cessation of studying, the external device may play an alarm sound or display, on a screen, a notification for stopping studying.

Thus, in the present embodiment, following operations can be executed based on information such as the user's concentration state: when the user is not concentrating, an operation for promoting concentration is executed; when the user is concentrating, an operation for maintaining concentration is executed; when fatigue due to a long period of state of concentration or extended study time is observed, or when it is predicted that fatigue will remain, an operation for prompting a break may be executed. A method for prompting a break includes, for example, outputting sound, changing the screen display, or generating a scent from external devices such as a smartphone, a tablet, or a computer.

Here, with regard to the writing motion, not only a case of performing writing motion, but also a case of merely holding a writing instrument, such as thinking while holding the writing instrument, is included. That is, even when the user is merely holding the writing instrument (i.e., holding the writing instrument without performing writing motions), acceleration information distinctly different from that when not holding a writing instrument can be acquired. Therefore, the writing motion includes not only actual writing motions, but also motions and states from picking up the writing instrument to putting it down (writing-related motions), such as a state of pausing writing to think.

### (Other Configuration Examples)

Other configuration examples of the present disclosure include the following.

Other configuration examples of the electronic device 100 that records writing motions are listed below: a configuration where an acceleration sensor or a pressure sensor is present in the writing instrument itself; and a configuration where an acceleration sensor or a pressure sensor is present in a form attached to the writing instrument. In a case where the pressure sensor is provided, the pressure during writing (such as writing pressure or gripping pressure on the writing instrument) may be detected by the pressure sensor, and acceleration information may be acquired during a period when a pressure exceeding a predetermined threshold is detected. Furthermore, the biological indicator may be predicted based on the acceleration information during the period when a pressure exceeding the predetermined threshold is detected.

The analysis of writing motions and the prediction processing of the biological indicator are executed by any one of: the electronic device 100, the information processing device 200and a cloud computer on a network. Based on the analysis results, the biological indicator such as brain waves, heart rate, and respiration, and the human behaviors/human body movements (biological behavior indicators) such as blinking and facial movements may be predicted. In the case of brain wave, concentration, contemplation, relaxation, drowsiness, and sleep and the like may be predicted. In the case of heart rate, comfort/discomfort, anger, and pleasure and the like may be predicted.

Based on the predicted biological indicators and human behaviors/human body movements, the electronic device 100 or the information processing device 200 may present sounds, music, or screen displays such as text, colors, or illustrations, thereby prompting behavior changes in the person, such as enabling increased concentration, enhanced relaxation, awakening, or affording pleasure. The information processing device 200 may include an output generation unit configured to generate output information for prompting the aforementioned behavior change.

### (Verification of the Prediction Model)

During construction of a concentration prediction model based on the writing motion, brain waves, heart rate, and electromyograms, etc. were acquired at the Graduate School of Pharmaceutical Sciences, The University of Tokyo, using a DL-160A electroencephalograph, a DL-310 heart rate monitor, and an SX230 electromyograph, all manufactured by Q'sfix.

For the writing data, an electronic device attached to a writing instrument was used. This electronic device is equipped with an acceleration sensor, memory, a Bluetooth^{®} chip, a USB terminal, and other components, and is capable of acquiring information on the movement of the writing instrument/ information on writing motions via the acceleration sensor and transmitting the information to an external device, such as a smartphone or tablet. In addition, this electronic device may be integrated with the writing instrument. Acceleration is acquired at an acquisition frequency in a range of approximately 0.1 Hz to 1 kHz.

Electrodes for acquiring brain waves, heart rate, and electromyography are attached to a subject (user). The subject (user) uses the electronic device 100, which is attached to the writing instrument, and measures the brain waves at the time of performing writing motions are measured under various conditions using an electroencephalograph. In addition, during this experiment, the subject (user) is also recorded with a video camera to record head movements, eye movements, blinks, and the like on the video camera for subsequent analysis.

As the experiment method, the subject is connected to measuring devices, such as an electroencephalograph, a heart rate monitor, and an electromyography device. While being recorded with a video camera and simultaneously measuring brain waves, heart rate, and electromyographic signals, the subject performs the writing motion using the electronic device 100 attached to the writing instrument as an attachment, with acceleration data acquired from the attachment on the writing instrument.

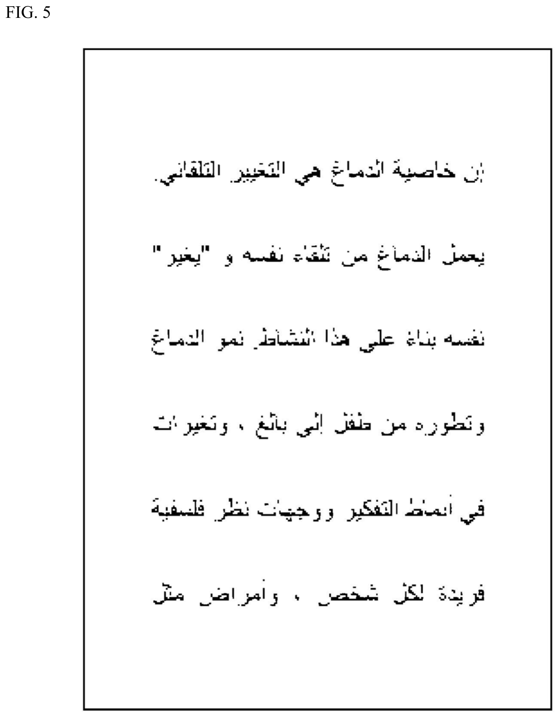

At this time, the following writing motions are performed:
(1) Write Arabic characters as shown in FIG. 5. It is noted that although Arabic characters were used in this experiment, what is written is not limited to Arabic characters.
(2) A movie is played beside the subject while he/she is writing, and the subject writes Arabic characters while watching the movie.
(3) Write the Arabic characters while engaging in a conversation with another person.
(4) Write the Arabic characters with full concentration without engaging in any distracting activities.
(5) Even though a movie is being played beside the subject while he/she is writing, the subject is instructed not to watch the movie and to write Arabic characters.
(6) Write the Arabic characters while focusing on a conversation with another person.
(7) Draw a picture.
(8) Perform calculations.
(9) Solve a logic quiz.
(10) Solve calculation problems such as an SPI (Synthetic Personality Inventory) test.

Writing while watching a movie and writing while engaging in a conversation intentionally create states in which concentration is not maintained. Each of the above is performed for 10 minutes.

Through these experimental settings, it is possible to acquire data under various scenarios including actual writing and holding the writing instrument while engaging in a conversation.

Regarding the brain waves, a Fourier transform is performed, and a 10-second average is calculated for a y wave and a δ-wave, excluding any obvious outliers (values exceeding 3σ are excluded). This is intended to eliminate abnormal measurement values measured by the brain wave measurement device. In addition to the 10-second average, a 1-minute average and other related data were also calculated.

A learning model is established using deep learning techniques based on the brain waves and acceleration movements. In this experiment, a sequence-to-one regression long short-term memory (LSTM) network is used to establish the learning model. When creating and validating a learning model for a same person, the data are divided in an 8:1:1 ratio and respectively used as training data (train dataset) for model creation, validating data (validation dataset) for evaluating the adequacy of parameters, and testing data (test dataset) for assessing performance.

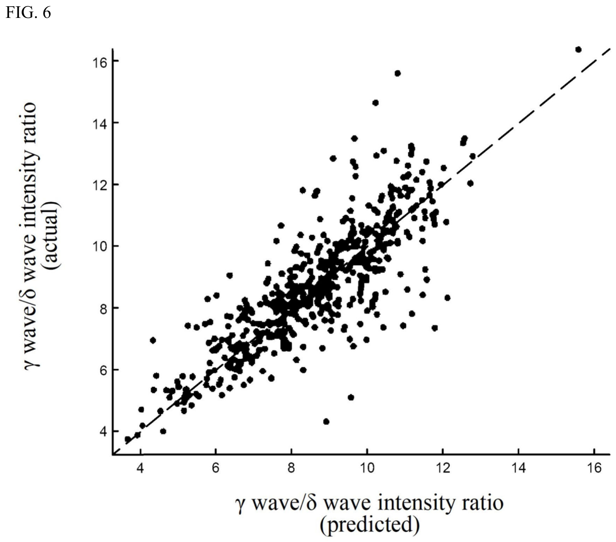

As a result, as shown in FIG. 6, a strong correlation is observed between brain waves (γ-wave/δ-wave) predicted from the writing motion (acceleration data) and the actually measured brain waves (γ-wave/δ-wave), indicating that brain waves can be predicted from the acceleration data of the attachment mounted on the writing instrument. A horizontal axis represents the brain wave data predicted from the acceleration, and a vertical axis represents the actually measured brain wave data. In this experiment, the γ wave/δ wave, that is, the value obtained by dividing the γ wave by the δ wave, is used in order to normalize variations in brain waves across individuals, time, and intensities.

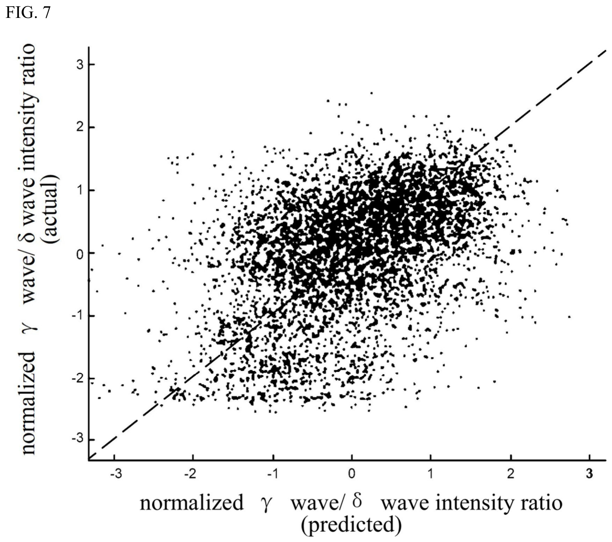

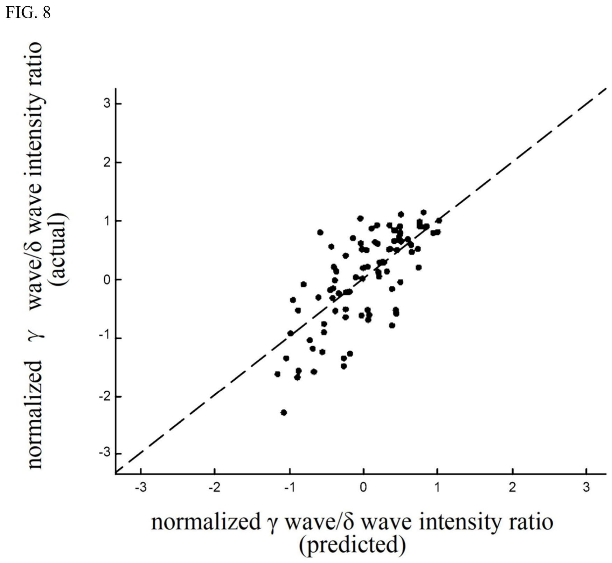

Furthermore, brain waves are predicted not only for the subject (1) but also for subjects other than subject (1). The brain wave and writing data of six subjects (Subjects (1) to (6)) are used as training data to establish a prediction/learning model, and brain waves of Subject (7) are predicted from acceleration data of Subject (7)'s writing motion. The results are shown in FIG. 7, where the horizontal axis represents brain wave data predicted from acceleration, and the vertical axis represents actually measured brain wave data. The correlation coefficient in this case is 0.4396, indicating that there is a correlation. This Data is a prediction result predicted at 10-second intervals (10-second average), but when the learning model is established and predictions are made at 1-minute intervals (1-minute averages), the results will be as shown in FIG. 8. In this case, the correlation coefficient is 0.7298, which is good.

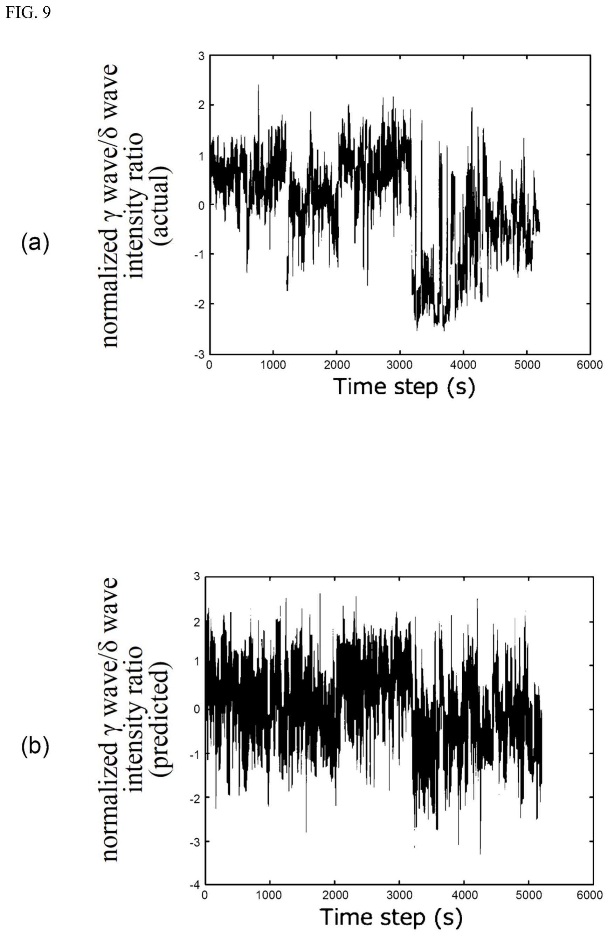

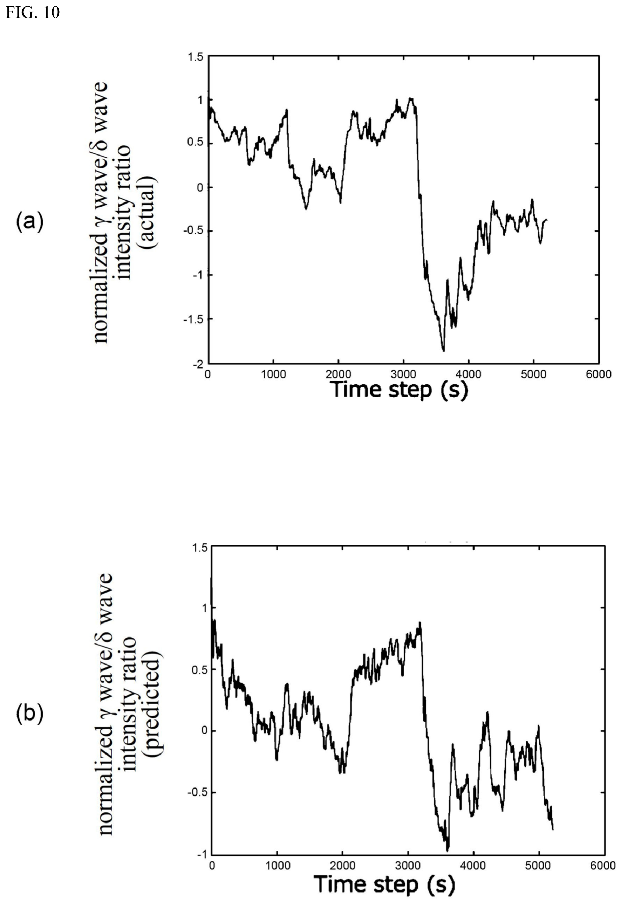

FIGS. 9 (A) and 9 (B) illustrate a comparison between brain waves predicted from the writing motions based on the 10-second interval data shown in FIG. 7 and the actually measured brain waves. FIG. 9 A shows the actually measured brain waves (γ-wave/δ-wave), and FIG. 9(B) shows brain waves (γ-wave/δ-wave) predicted from the writing motions (acceleration data). In addition, FIGS. 10(A) and 10(B) illustrate a comparison between brain waves predicted from the writing motions based on the 1-minute interval data shown in FIG. 8 and the actually measured brain waves. FIG. 10(A) shows the actually measured brain waves (γ-wave/δ-wave), and FIG. 10(B) shows brain waves (γ-wave/δ-wave) predicted from the writing motions (acceleration data). In each of FIGS. 9(A), 9(B) and FIGS. 10(A), 10(B), the horizontal axis represents time, and the vertical axis represents brain waves (γ-wave/δ-wave). It can be seen that there is a good agreement.

### (Regarding Feedback)

The brain waves predicted from the writing motions as described above may be fed back to the user using, for example, the following methods. When the intensity of the brain waves (y waves) is high, indicating that the user is in a state of concentration, the external device may be set to silent or play soft sounds to help maintain the user's concentration. Conversely, when the brain waves are weak, the external device may play awakening music or change the screen display to text, illustrations, colors, photos, or the like that promote concentration. The feedback provided to the user may be based not only on the predicted brain waves but also on the actual brain waves.

### (Regarding Measurement of Brain Waves and the Like)

Regarding the device for measuring brain waves, any general electroencephalograph may be employed, regardless of whether it is of the dry type or the wet type. As for the device for measuring heart rate, a medical electrocardiograph, a wristband-type mobile device, or a smart watch may be employed.

In this experiment, university students are subjects for constructing the learning model of brain waves and writing motions. However, any individual capable of writing or drawing may serve as a subject, including persons ranging from young children to elderly individuals.

### (Regarding Deployment of Service and System)

Environments for deploying a service and system implementing the information processing system 1, the information processing apparatus 300, and the information processing method according to the present disclosure include, for example, homes, cram schools, study rooms, cafés, and schools. When sound is played in environments where other individuals are present, it may be combined with an external device capable of delivering sound to individual, such as headphones.

### (Regarding Learning Model)

In this experiment, the learning model was established using a deep learning sequence-to-one regression LSTM network. However, the learning model is not limited thereto, and other machine learning or deep learning techniques, such as a Transformer, may also be employed.

### (Regarding Brain Waves)

Regarding brain waves, in this experiment, a normalized metric of γ-wave/δ-wave (y-wave divided by δ-wave) was used. However, it is not limiting, and any method for measuring or predicting the occurrence of brain waves within a frequency range of approximately 0.1 Hz to 100 Hz may also be employed. Generally, frequencies known as γ-wave, δ-wave, or high α-wave often represent a state of concentration. Further, θ-wave represents a state of relaxation or sleep. Other brain such as α-waves and β-waves are also emitted.

It is also possible to identify the relationship between these brain waves and the writing motions. Furthermore, because brain wave intensity varies between individuals, it is also possible to obtain a normalized value by dividing the brain wave of a specific frequency by a brain wave of another frequency.

The state generally referred to as concentration refers to a state of high cognitive load or a state of hard thinking.

### (Regarding Other System Configurations)

In the embodiments described above, examples have been provided in which the acceleration sensor is provided in an electronic device 100, either in the form of the attachment mounted on the writing instrument or integrated with the writing instrument. However, the method of identifying brain waves from writing motions is not limited thereto. For example, when writing directly on a tablet, acceleration information may be obtained by using the written strokes or trajectory, by using an acceleration sensor mounted on a stylus pen, or by using both.

Similarly, in Virtual Reality (VR), Augmented Reality (AR), or Mixed Reality (MR) environments, when writing motions are performed at predetermined spatial coordinates, acceleration information of the pen stroke or trajectory of the writing action may be read using information from a controller or camera, and the level of concentration may be determined. For example, an acceleration component along a specific plane at predetermined spatial coordinates (including a region within a predetermined tolerance in the direction orthogonal to the plane) may be read as acceleration information, and the level of concentration may be determined based on such information.

### (Regarding Application)

An application used on a smartphone, tablet, or computer is configured to include at least one of the following functions:
- A function of receiving writing motions/ information on writing motions, such as acceleration data.
- A function of processing the acceleration data and other information using machine learning, deep learning, AI, etc. to predict/determine biological information and behaviors, such as brain waves.
- A function of comparing the predicted/ determined results with a preset numeric value table.
- A function of performing predetermined outputs, such as producing a sound, displaying or changing screen display content, or emitting scent, according to the comparison results.

The information processing program (application software) according to the present disclosure has following configurations.

An information processing program executed by an information processing computer causes the information processing computer to perform: an acceleration acquisition step, which acquires information regarding acceleration during a writing motion; and a prediction processing step, which outputs information related to the prediction of a biological indicator or a biological behavior of a user based on a pre-established artificial intelligence learning model using the information regarding the acceleration detected in the acceleration acquisition step.

The information processing program is recorded on a storage medium or transmitted via a network.

### (Regarding Acquisition of Writing Motions)

Acquisition of writing motions can be performed not only by electrifying the writing instrument itself or attaching an attachment to the writing instrument, but also by the following methods.
- A method of attaching a sensing device such as an acceleration sensor to arm, such as a ring-type or wristband-type device.
- A method of capturing images with a camera to determine the writing motions.

### (Regarding Behavioral Indicators)

In the above-described experiment, when the subjects/users performed the writing motions (1) to (10) under brain wave measurement, the subjects/users were recorded by a video camera and actions that serve as behavioral indicators, such as head movements and blinking, were captured. Accordingly, by an artificial intelligence employing the correlation between acceleration information in the writing motions and the behavioral indicators of the user as a learning model, the behavioral indicators representing the user's concentration, such as head movements, eye movements, and blinking, from the acceleration information during the writing motions can be predicted.

As described above, according to the present embodiment, it is possible to provide an information processing system and an information processing method that predict biological indicators, such as brain waves and heart rate, as well as biological behaviors, such as eye movements and blinking, based on writing motions.

It should be understood that although the embodiments of the present disclosure and of their application examples have been described above, the present disclosure is not limited to these embodiments or examples. For example, for each of the above embodiments or their application examples, appropriate additions, deletion, or design change of the components, or appropriate combinations of features of the respective embodiments made by those skilled in the art are also encompassed within the scope of the present invention, as long as the gist of the present invention is retained.

### EXPLANATION OF REFERENCE SYMBOLS

- 1: Information Processing System
- 100: Electronic Device
- 110: Main Body
- 120: Acceleration Detection Unit
- 130: Communication Unit
- 131: Transmission Unit
- 132: Reception Unit
- 140: Memory
- 150: Input Unit
- 160: Output Unit
- 170: Power Supply
- 180: Control Unit
- 200: Information Processing Device
- 210: Communication Unit
- 211: Transmission Unit
- 212: Reception Unit
- 220: Prediction Processing Unit
- 240: Memory
- 250: Input Unit
- 260: Output Unit
- 270: Power Supply
- 280: Control Unit
- 300: Information Processing Apparatus
- 310: Acceleration Detection Unit
- 320: Prediction Processing Unit

## Claims

1. An information processing system comprising an electronic device and an information processing device, wherein
the electronic device comprises;
a main body configured to be a pen-shaped main body with a writing function or configured to be attachable to a writing instrument;
an acceleration detection unit configured to detect information regarding acceleration during a writing motion;
a transmission unit configured to transmit the information regarding the acceleration detected by the acceleration detection unit to the information processing device;
the information processing device comprises:
a reception unit configured to receive the information regarding the acceleration transmitted from the transmission unit;
a prediction processing unit configured to use the information regarding the acceleration received by the reception unit as input, and to output information regarding a prediction for a biological indicator or a biological behavior of a user based on a pre-established learning model of artificial intelligence.

2. The information processing system according to claim 1, wherein the prediction processing unit uses the learning model based on a correlation between a temporal variation pattern of the acceleration during the writing motion and the biological indicator or the biological behavior, wherein the biological indicator or the biological behavior represents any one of a degree of concentration, a degree of relaxation, and a degree of comfort of the user performing the writing motion.

3. The information processing system according to claim 1, wherein the prediction processing unit uses the learning model based on a correlation between the acceleration and brain waves during the writing motion.

4. The information processing system according to claim 3, wherein the prediction processing unit is configured to predict occurrence of the brain waves a frequency in a range of 0.1 Hz to 100 Hz.

5. The information processing system according to claim 1, wherein the information regarding the acceleration comprises acceleration along a writing trajectory or an acceleration component along a specific plane at predetermined spatial coordinates.

6. The information processing system according to claim 1, wherein the electronic device further comprises a pressure detection unit configured to detect pressure during writing,
the information regarding the acceleration comprises information of the acceleration during a period in which the pressure exceeding a predetermined threshold is detected by the pressure detection unit.

7. The information processing system according to claim 1, wherein the information processing device further comprises an output generation unit configured to generate predetermined output information based on the biological indicator or the biological behavior predicted by the prediction processing unit.

8. An information processing apparatus, comprising:
an acceleration detection unit configured to detect information regarding acceleration during a writing motion; and
a prediction processing unit configured to use the information regarding the acceleration detected by the acceleration detection unit as input, and to output information regarding a prediction for a biological indicator or a biological behavior of a user based on a pre-established learning model of artificial intelligence.

9. The information processing apparatus according to claim 8, wherein the prediction processing unit uses the learning model based on a correlation between a temporal variation pattern of the acceleration during the writing motion and the biological indicator or the biological behavior, wherein the biological indicator or the biological behavior represents any one of a degree of concentration, a degree of relaxation, and a degree of comfort of the user performing the writing motion.

10. The information processing apparatus according to claim 8, wherein the prediction processing unit uses the learning model based on a correlation between the acceleration and brain waves during the writing motion.

11. The information processing apparatus according to claim 10, wherein the prediction processing unit is configured to predict occurrence of the brain waves of a frequency in a range of 0.1 Hz to 100 Hz.

12. The information processing apparatus according to claim 8, wherein the information regarding the acceleration comprises acceleration along a writing trajectory or an acceleration component along a specific plane at predetermined spatial coordinates.

13. The information processing apparatus according to claim 8, further comprising a pressure detection unit configured to detect pressure during writing, wherein
the information regarding the acceleration comprises information of the acceleration during a period in which the pressure exceeding a predetermined threshold is detected by the pressure detection unit.

14. The information processing apparatus according to claim 8, further comprising an output generation unit configured to generate predetermined output information based on the biological indicator or the biological behavior predicted by the prediction processing unit.

15. An information processing method, comprising:
an acceleration acquisition step of acquiring information regarding acceleration during a writing motion; and
a prediction processing step of outputting information regarding a prediction for a biological indicator or a biological behavior of a user based on a pre-established learning model of artificial intelligence from the information regarding the acceleration detected in the acceleration acquisition step.

16. The information processing method according to claim 15, wherein the prediction processing step comprises using the learning model based on a correlation between a temporal variation pattern of the acceleration during the writing motion and the biological indicator or the biological behavior, wherein the biological indicator or the biological behavior represents any one of a degree of concentration, a degree of relaxation, and a degree of comfort of the user performing the writing motion.

17. The information processing method according to claim 15, wherein the prediction processing step comprises using the learning model based on a correlation between the acceleration and brain waves during the writing motion.

18. The information processing method according to claim 17, wherein the prediction processing step comprises predicting an occurrence of the brain waves of a frequency in a range of 0.1 Hz to 100 Hz.

19. The information processing method according to claim 15, wherein the information regarding the acceleration comprises acceleration along a writing trajectory or an acceleration component along a specific plane at predetermined spatial coordinates.

20. The information processing method according to claim 15, further comprising:
a pressure acquisition step of acquiring pressure during writing, wherein
the information regarding the acceleration comprises information of the acceleration during a period in which the pressure of a predetermined value is acquired in the pressure acquisition step.

21. The information processing method according to claim 15, further comprising an output generation step of generating predetermined output information based on the biological indicator or the biological behavior predicted in the prediction processing step.
